# EUROPÄISCHE PATENTANMELDUNG

(11) **EP 3 006 015 A1**
(43) Veröffentlichungstag der Anmeldung: **13.04.2016**
(21) Anmeldenummer: 15193631.7
(22) Anmeldetag: 21.06.2005
(51) Int. Cl.: A61K 8/35, A61Q 19/06, A61Q 19/08, A61Q 17/00

(54) **VERWENDUNG VON WIRKSTOFFKOMBINATIONEN AUS EINEM ODER MEHREREN BIOCHINONEN UND EINEM ODER MEHREREN ISOFLAVONEN ZUR VERBESSERUNG DER HAUTKONTUREN**

(30) Priorität: 02.07.2004 DE 102004032837
(62) Teilanmeldung aus: 05013302.4
(71) Anmelder: Beiersdorf AG, 20253 Hamburg (DE)
(72) Erfinder: MIELKE, Heiko, 21629 Neu Wulmstorf (DE); JAPSERS, Sören, 22869 Schenefeld (DE); HAMER, Gundhild, 22455 Hamburg (DE); MUMMERT, Christopher, 29553 Bienenbüttel (DE); SCHULZ, Jens, 25462 Rellingen (DE); GALLINAT, Stephan, 22880 Wedel (DE); VENZKE, Kirsten, 26725 Emden (DE); SCHWANKE, Frank, 22527 Hamburg (DE)

(57) **Zusammenfassung**

Verwendung kosmetischer oder dermatologischer Zubereitungen in Form von O/W-Emulsionen mit einem Gehalt an
(a) einem oder mehreren Biochinonen und
(a) einem oder mehreren Isoflavonoiden

zur Straffung und/oder Festigung der Haut bzw. zur kosmetischen und/oder dermatologischen Behandlung und/oder Prophylaxe der Cellulite.

## Beschreibung

Verwendung von Wirkstoffkombinationen aus einem oder mehreren Biochinonen und einem oder mehreren Isoflavonen zur Verbesserung der Hautkonturen

Die vorliegende Erfindung betrifft kosmetische bzw. dermatologische dermatologische Zubereitungen, enthaltend Wirkstoffe zur Pflege und zum Schutze der Haut, insbesondere der empfindlichen Haut wie auch ganz besonders im Vordergrunde stehend der durch intrinsische und/oder extrinsische Faktoren gealterten oder alternden Haut sowie die Verwendung solcher Wirkstoffe und Kombinationen solcher Wirkstoffe auf dem Gebiete der kosmetischen und dermatologischen Hautpflege.

Unter kosmetischer Hautpflege ist in erster Linie zu verstehen, dass die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Die chronologische Hautalterung wird z.B. durch endogene, genetisch determinierte Faktoren verursacht. In Epidermis und Dermis kommt es alterungsbedingt z.B. zu folgenden Strukturschäden und Funktionsstörungen, die auch unter den Begriff "Senile Xerosis" fallen können:
a) Trockenheit, Rauhigkeit und Ausbildung von Trockenheitsfältchen,
b) Juckreiz und
c) verminderte Rückfettung durch Talgdrüsen (z.B. nach Waschen).

Exogene Faktoren, wie UV-Licht und chemische Noxen, können kumulativ wirksam sein und z.B. die endogenen Alterungsprozesse beschleunigen bzw. sie ergänzen. In Epidermis und Dermis kommt es insbesondere durch exogene Faktoren z.B. zu folgenden Strukturschäden- und Funktionsstörungen in der Haut, die über Maß und Qualität der Schäden bei chronologischer Alterung hinausgehen:
d) Sichtbare Gefäßerweiterungen (Teleangiektasien, Cuperosis);
e) Schlaffheit und Ausbildung von Falten;
f) lokale Hyper-, Hypo- und Fehlpigmentierungen (z.B. Altersflecken) und
g) vergrößerte Anfälligkeit gegenüber mechanischem Stress (z.B. Rissigkeit).

Die vorliegende Erfindung betrifft insbesondere Produkte zur Pflege der auf natürliche Weise gealterten Haut, sowie der unter a), e) und g) aufgeführten Phänomene.

Produkte zur Pflege erschlaffter, insbesondere gealterter Haut sind an sich bekannt. Sie enthalten z.B. Retinoide (Vitamin A-Säure und/oder deren Derivate) bzw. Vitamin A und/oder dessen Derivate. Ihre Wirkung auf die Strukturschäden ist allerdings umfangsmäßig begrenzt. Darüber hinaus gibt es bei der Produktentwicklung erhebliche Schwierigkeiten, die Wirkstoffe in ausreichendem Maße gegen oxidativen Zerfall zu stabilisieren. Die Verwendung Vitamin A-Säure-haltiger Produkte bedingt darüber hinaus oft starke erythematöse Hautreizungen. Retinoide sind daher nur in geringen Konzentrationen einsetzbar.

Oftmals wird mit der erschlafften Haut auch eine Begleiterscheinung der Übergewichtigkeit und/oder der damit häufig einhergehenden so genannten Cellulite verbunden.

Das Körperbewusstsein der Verbraucher ist in den vergangenen Jahren deutlich gestiegen. Dabei werden neben reinigenden und pflegenden Anwendungen auch zunehmend Maßnahmen ergriffen, um die Körpersilhouette zu verbessern. Die Cellulite - ein weit verbreitetes Phänomen - nimmt dabei eine zentrale Stellung ein. Das sichtbare Bild der Cellulite beruht auf einer Zunahme von Fettpolstern in der Subcutis (Unterhautfettgewebe), einer Bindegewebsschwäche sowie einer Minderung der Durchströmungsverhältnisse in den Blut- und Lymphbahnen. Die Ursache ist somit eine zum Teil anlagebedingte Schwächung des Bindegewebes mit gleichzeitigem Auftreten von vergrößerten Fettzellkammern infolge von Übergewicht, unausgewogener Ernährung, Bewegungsmangel. Die Entstehung der Cellulite kann ferner auf eine erhöhte Durchlässigkeit der Haargefäßwände zurückgeführt werden, die das Eindringen von Wasser ins Bindegewebe erlaubt.

Daneben kann an den betroffenen Hautbereichen ein lokaler Mangel an Testosteron bestehen. Jedenfalls ist Cellulite eine Erscheinung die fast niemals bei Männern zu beobachten ist.

Aufgabe der vorliegenden Erfindung war es somit, Wege zu finden, die die Nachteile des Standes der Technik vermeiden. Insbesondere war es eine Aufgabe der vorliegenden Erfindung, Zubereitungen zur Verfügung zu stellen, die eine vorteilhafte Straffung erschlaffter Haut bewirken können.

Es hat sich überraschenderweise herausgestellt, dass die Verwendung kosmetischer oder dermatologischer Zubereitungen in Form von O/W-Emulsionen mit einem Gehalt an
(a) einem oder mehreren Biochinonen und
(a) einem oder mehreren Isoflavonoiden
zur Straffung und/oder Festigung der Haut den Nachteilen des Standes der Technik abhilft.

Erfindungsgemäß vorteilhaft ist weiterhin die Verwendung kosmetischer oder dermatologischer Zubereitungen in Form von O/W-Emulsionen mit einem Gehalt an
(a) einem oder mehreren Biochinonen und
(a) einem oder mehreren Isoflavonoiden
zur kosmetischen und/oder dermatologischen Behandlung und/oder Prophylaxe der Cellulite.

Ubichinone zeichnen sich durch die Strukturformel 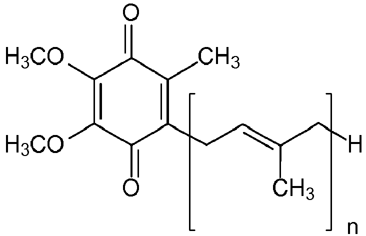 aus und stellen die am weitesten verbreiteten u. damit am besten untersuchten Biochinone dar. Ubichinone werden je nach Zahl der in der Seitenkette verknüpften Isopren-Einheiten als Q-1, Q-2, Q-3 usw. oder nach Anzahl der C-Atome als U-5, U-10, U-15 usw. bezeichnet. Sie treten bevorzugt mit bestimmten Kettenlängen auf, z. B. in einigen Mikroorganismen u. Hefen mit n=6. Bei den meisten Säugetieren einschließlich des Menschen überwiegt Q10.

Coenzym Q10 beispielsweise ist durch folgende Strukturformel gekennzeichnet:

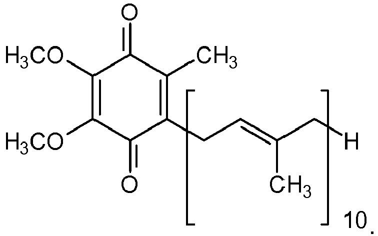

Ubichinone dienen den Organismen als Elektronenüberträger in der Atmungskette. Sie befinden sich in den Mitochondrien wo sie die cyclische Oxidation und Reduktion der Substrate des Citronensäure-Cyclus ermöglichen.

Plastochinone weisen die allgemeine Strukturformel 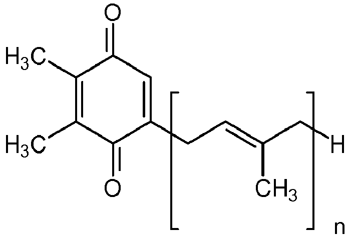 auf. Sie können aus Chloroplasten isoliert werden und spielen als Redoxsubstrate in der Photosynthese beim cyclischen und nichtcyclischen Elektronentransport eine Rolle, wobei sie reversibel in die entsprechenden Hydrochinone (Plastochinol) übergehen. Plastoschinone unterscheiden sich in der Anzahl n der Isopren-Reste und werden endsprechend bezeichnet, z. B. PQ-9 (n=9). Ferner existieren andere Plastochinone mit unterschiedlichen Substituenten am Chinon-Ring.

In der japanischen Offenlegungsschrift 58,180,410 ist die Eignung von Coenzym Q10 für Kosmetika beschrieben. Es soll den Hautzellmetabolismus aktivieren und die Oxidation unterdrücken. Coenzym Q10 hat im Resultat eine wichtige Funktion bei der Prävention von Hautschäden durch UV-Strahlen und der Prävention von Hautalterung.

Die Internationalen Patentanmeldungsschriften WO95/26180, WO95/26181 und WO95/26182 betreffen kosmetische und dermatologische Zubereitungen mit einem Gehalt an Ubichinonen bzw. Wirkstoffkombinationen mit Ubichinonen, wobei diese Zubereitungen auch gegen senile Xerosis und Hautalterung eingesetzt werden können. Diese Zubereitungen liegen zwar auch vereinzelt als O/W-Emulsionen vor, jedoch mit einem deutlich niedrigeren Gehalt an Glycerin als es im Sinne der hiermit vorgelegten technischen Lehre von Vorteil ist.

Kosmetische Zubereitungen mit Coenzym Q10 sind ferner aus der DE-A-33 09 850 bekannt, die zur Behandlung von Hautkrankheiten, zur Prophylaxe von dystrophischen und dysmetabolischen Zuständen der Haut und zur Anwendung bei chemischen und physikalischen Respirationsschäden oder bei verzögerter Respiration verbunden mit Alter und Abnutzung geeignet sind.

All diese Dokumente konnten jedoch nicht den Weg zur vorliegenden Erfindung ebnen.

Erfindungsgemäß bevorzugtes Biochinon ist das Coenzym Q10. Es ist vorteilhaft, in den fertigen Zubereitungen Konzentrationen von 0,000.001 - 5 Gew.-% an einem oder mehreren Biochinonen, bevorzugt Coenzym Q10, zu wählen, bezogen auf das Gesamtgewicht der Zubereitungen.

Isoflavone sind zu den Flavonoiden zählende, gelegentlich auch als Isoflavonoide bezeichnete Gruppe von meist gelblich gefärbten Pflanzenfarbstoffen, die sich von Isoflavon ableiten. Der unsubstituierte Grundkörper, das eigentliche Isoflavon (3-Phenylchromon, 3-Phenyl-4H-1-benzopyran-4-on) kommt in Kleearten vor.

Einige bekanntere Isoflavone sind Daidzein (4',7-Dihydroxy-Isoflavon), als 7-O-Glucosid Daidzin in Sojamehl; Genistein (4',5,7-Trihydroxy-Isoflavon) aus Sojabohnen und Rotklee; Prunetin (4',5-Dihydroxy-7-methoxy-Isoflavon) aus der Rinde von Pflaumenbäumen; Biochanin A (5,7-Dihydroxy-4'-methoxy-Isoflavon) aus Kichererbsen, Rotklee und anderen Kleearten; Orobol (3',4',5,7-Tetrahydroxy-Isoflavon); Santal (3',4',5-Trihydroxy-7-methoxy-Isoflavon) aus Sandelholz, Rotholz und anderen Hölzern; Pratenseïn (3',5,7-Trihydroxy-4'-methoxyisoflavon) aus frischem Rot- oder Wiesenklee. Einige dieser in Kleearten und Leguminosen wie Luzerne vorkommenden Isoflavone zeigen Östrogenwirkung auf Weidetiere und können unter umständen zu Fortpflanzungsstörungen bei diesen führen.

Im Folgenden sind Substitutionsschemata einiger natürlich vorkommender Isoflavone aufgefü h rt:

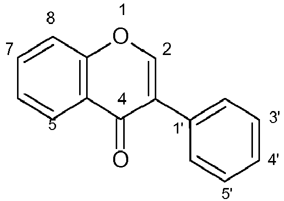

| | 5 | 7 | 3' | 4' | CAS-Nr. |
|---|---|---|---|---|---|
| Isoflavon | H | H | H | H | 574-12-9 |
| Daidzeïn | H | OH | H | OH | 486-68-8 |
| Genistein | OH | OH | H | OH | 446-72-0 |
| Genistin | OH | OH | H | OH | 446-72-0 |
| Prunetin | OH | OCH₃ | H | OH | 552-59-0 |
| Biochanin A | OH | OH | H | OCH₃ | 491-80-5 |
| Orobol | OH | OH | OH | OH | 480-23-9 |
| Santal | OH | OCH₃ | OH | OH | 529-60-2 |
| Pratenseïn | OH | OH | OH | OCH₃ | 2284-31-3 |

Als erfindungsgemäß einzusetzende Isoflavone kommen vorzugsweise die in der vorstehenden Tabelle aufgeführten Isoflavone in Betracht.

Von diesen wiederum ist bevorzugt das Genistein.

Genistein, 5,7-Dihydroxy-3-(4-hydroxyphenyl)-4*H*-1-benzopyran-4-on, 4',5,7-Trihydroxyisoflavon, auch Differenol A, Prunetol und Sophoricol genannt. weist folgende Struktur auf:

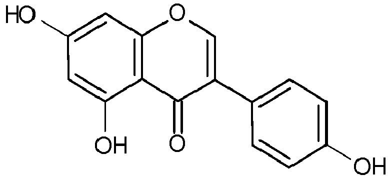

Genistein ist ein Sekundärmetabolit aus Pflanzen (Leguminosen, Papilionoiden, Rosaceen), wurde jedoch auch in Kulturen von Mikroorganismen (Actinomyceten, Aspergillus, Mycobacterien) gefunden. Es wurden schwach östrogene und antibakterielle Wirkungen beschrieben.

Bevorzugt enthalten kosmetische oder dermatologische Zubereitungen gemäß der Erfindung 0,001 - 10 Gew.-%, besonders bevorzugt 0,01 - 1 Gew.-%, an einem oder mehreren Isoflavonoiden, bezogen auf die Gesamtzusammensetzung der Zubereitungen.

Bevorzugt werden die Gewichtsverhältnisse von einem oder mehreren Isoflavonoiden zu einem oder mehreren Biochinonen in den Wirkstoffkombinationen und Zubereitungen, solche Wirkstoffzubereitungen enthaltend, gewählt aus dem Bereich von 50 : 1 bis 1 : 50, vorteilhaft aus dem Bereich von 10 : 1 bis 1 : 50, insbesondere vorteilhaft 2 : 1 bis 1 :2.

Insbesondere ist es dabei vorteilhaft, wenn sich diese Extrakte zusätzlich durch einen Gehalt an Saponinen auszeichnen, da die zu erzielenden Wirkungen in diesem Fall besonders ausgeprägt sind. Der Gehalt an Saponinen sollte dabei in einem Konzentrationsbereich von 0.001 - 2 Gew.-%, bevorzugt 0,02 - 0.04 Gew.-%, bezogen auf die Gesamtzusammensetzung der kosmetischen Zubereitung, liegen.

Dabei kann eine vorteilhafte Ausführungsform der vorliegenden Erfindung darin bestehen, dass das oder die Isoflavonoide als Isoflavonoid-haltiger Extrakt in Form eines Soja-Extraktes eingesetzt wird oder werden, der neben Isoflavonoiden, bevorzugt Genistein, 5 - 20 Gew.-% Saponine, besonders vorteilhaft 10 - 18 Gew.-% Saponine, jeweils bezogen auf das Gesamtgewicht des Extraktes, enthält.

Die Anwendung dieser Zubereitungen führt zu einer Verbesserung der Hautkonturen und zu einer erhöhten Elastizität, insbesondere an den bindegewebsschwachen Arealen des Körpers wie z.B. an den Beinen, am Bauch und am Gesäß. Diese hautstraffenden und - festigenden Wirkungen gehen über eine Verbesserung des Zellstoffwechsels mit der Reduzierung der Orangenhaut einher, also beispielsweise mit der Verminderung von Hautdellen am Oberschenkelbereich ("Reiterhosen").

Es ist erfindungsgemäß insbesondere äußerst vorteilhaft, den erfindungsgemäß verwendeten Wirkstoff bzw. kosmetische oder topische dermatologische Zubereitungen mit einem wirksamen Gehalt an erfindungsgemäß verwendetem Wirkstoff zur kosmetischen oder dermatologischen Behandlung oder Prophylaxe unerwünschter Hautzustände zu verwenden.

Erfindungsgemäß können Zubereitungen, welche die erfindungsgemäßen Wirkstoffkombinationen enthalten, übliche Antioxidantien eingesetzt werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Alanindiessigsäure, Flavonoide, Polyphenole, Catechine, Vitamin C und Derivate (z.B. Ascorbylpalmitat, Mg-Ascorbylphosphat, Ascorbylacetat), Tocopherole und Derivate (z.B. Vitamin-E-acetat), sowie Koniferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Die Prophylaxe bzw. die kosmetische oder dermatologische Behandlung mit dem erfindungsgemäß verwendeten Wirkstoff bzw. mit den kosmetischen oder topischen dermatologischen Zubereitungen mit einem wirksamen Gehalt an erfindungsgemäß verwendetem Wirkstoff erfolgt in der üblichen Weise, und zwar dergestalt, dass der erfindungsgemäß verwendete Wirkstoff bzw. die kosmetischen oder topischen dermatologischen Zubereitungen mit einem wirksamen Gehalt an erfindungsgemäß verwendetem Wirkstoff auf die betroffenen Hautstellen aufgetragen wird.

Erfindungsgemäße Emulsionen im Sinne der vorliegenden Erfindung, z.B. in Form einer Creme, einer Lotion, einer kosmetischen Milch sind vorteilhaft und enthalten z.B. Fette, Öle, Wachse und/oder andere Fettkörper, sowie Wasser und einen oder mehrere Emulgatoren, wie sie üblicherweise für einen solchen Typ der Formulierung verwendet werden.

Es ist auch möglich und vorteilhaft im Sinne der vorliegenden Erfindung, den erfindungsgemäß verwendeten Wirkstoff in wässrige Systeme bzw. Tensidzubereitungen zur Reinigung der Haut und der Haare einzufügen.

Es ist dem Fachmanne natürlich bekannt, dass anspruchsvolle kosmetische Zusammensetzungen zumeist nicht ohne die üblichen Hilfs- und Zusatzstoffe denkbar sind. Darunter zählen beispielsweise Konsistenzgeber, Füllstoffe, Parfum, Farbstoffe, Emulgatoren, zusätzliche Wirkstoffe wie Vitamine oder Proteine, Lichtschutzmittel, Stabilisatoren, Insektenrepellentien, Alkohol, Wasser, Salze, antimikrobiell, proteolytisch oder keratolytisch wirksame Substanzen usw.

Mutatis mutandis gelten entsprechende Anforderungen an die Formulierung medizinischer Zubereitungen.

Medizinische topische Zusammensetzungen im Sinne der vorliegenden Erfindung enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Es ist dabei ebenfalls von Vorteil, den erfindungsgemäß verwendeten Wirkstoff als Zusatzstoff zu Zubereitungen zu geben, die bereits andere Wirkstoffe für andere Zwecke enthalten.

Entsprechend können kosmetische oder topische dermatologische Zusammensetzungen im Sinne der vorliegenden Erfindung, je nach ihrem Aufbau, beispielsweise verwendet werden als Hautschutzcreme, Reinigungsmilch, Sonnenschutzlotion, Nährcreme, Tages- oder Nachtcreme usw. Es ist gegebenenfalls möglich und vorteilhaft, die erfindungsgemäßen Zusammensetzungen als Grundlage für pharmazeutische Formulierungen zu verwenden.

Es ist aber auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden beispielsweise in Tagescremes gewöhnlich UV-A- bzw. UV-B-Filtersubstanzen eingearbeitet.

Vorteilhaft können erfindungsgemäße Zubereitungen Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen.

Die UVB-Filter können öllöslich oder wasserlöslich sein. Als öllösliche Substanzen sind z.B. zu nennen:
- 3-Benzylidencampher und dessen Derivate, z.B. 3-(4-Methylbenzyliden)campher,
- 4-Aminobenzoesäure-Derivate, vorzugsweise 4-(Dimethylamino)-benzoesäure(2-ethylhexyl)ester, 4-(Dimethylamino)benzoesäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester;
- 2,4,6-Trianilino-(p-carbo-2'-ethyl-1'-hexyloxy)-1,3,5-triazin.

Als wasserlösliche Substanzen sind vorteilhaft:
- 2-Phenylbenzimidazol-5-sulfonsäure und deren Salze, z.B. Natrium-, Kalium- oder Triethanolammonium-Salze,
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Die Liste der genannten UVB-Filter, die erfindungsgemäß Verwendung finden können, soll selbstverständlich nicht limitierend sein.

Gegenstand der Erfindung ist auch die Kombination eines erfindungsgemäßen UVA-Filters mit einem UVB-Filter bzw. eine erfindungsgemäßes kosmetische oder dermatologische Zubereitung, welche auch einen UVB-Filter enthält.

Es kann auch von Vorteil sein, in erfindungsgemäßen Zubereitungen UVA-Filter einzusetzen, die üblicherweise in kosmetischen und/oder dermatologischen Zubereitungen enthalten sind. Bei solchen Filtersubstanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch Zubereitungen, die diese Kombinationen enthalten, sind Gegenstand der Erfindung. Es können die gleichen Mengen an UVA-Filtersubstanzen verwendet werden, welche für UVB-Filtersubstanzen genannt wurden.

Kosmetische und/oder dermatologische Zubereitungen im Sinne der vorliegenden Erfindung können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid. Es können die für die vorstehenden Kombinationen genannten Mengen verwendet werden.

Die erfindungsgemäßen kosmetischen und dermatologischen Zubereitungen können kosmetische Wirk-, Hilfs- und/oder Zusatzstoffe enthalten, wie sie üblicherweise in solchen Zubereitungen verwendet werden, z.B. Antioxidationsmittel, Konservierungsmittel, Bakterizide, Parfüme, Substanzen zum Verhindern des Schäumens, Farbstoffe, Pigmente, die färbende Wirkung haben, Verdickungsmittel, oberflächenaktive Substanzen, Emulgatoren, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Sofern die kosmetische oder dermatologische Zubereitung im Sinne der vorliegenden Erfindung eine Lösung oder Emulsion oder Dispersion darstellt, können als Lösungsmittel verwendet werden:
- Wasser oder wässrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkybenzoat und 2-Ethylhexylisostearat, Mischungen aus C₁₂₋₁₅-Alkybenzoat und Isotridecylisononanoat sowie Mischungen aus C₁₂₋₁₅-Alkybenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wässrige Phase der erfindungsgemäßen Zubereitungen enthält gegebenenfalls vorteilhaft
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Übliche Grundstoffe, welche für die Verwendung als kosmetische Stifte im Sinne der vorliegenden Erfindung geeignet sind, sind flüssige Öle (z.B. Paraffinöle, Ricinusöl, Isopropylmyristat), halbfeste Bestandteile (z.B. Vaseline, Lanolin), feste Bestandteile (z.B. Bienenwachs, Ceresin und Mikrokristalline Wachse bzw. Ozokerit) sowie hochschmelzende Wachse (z.B. Carnaubawachs, Candelillawachs)

Als Treibmittel für aus Aerosolbehältern versprühbare kosmetische und/oder dermatologische O/W-Emulsionen im Sinne der vorliegenden Erfindung sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, dass es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Kosmetische Zubereitungen im Sinne der vorliegenden Erfindung können auch als Gele vorliegen, die neben einem wirksamen Gehalt am erfindungsgemäßen Wirkstoff und dafür üblicherweise verwendeten Lösungsmitteln, bevorzugt Wasser, noch organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z. B. Aluminiumsilikate wie beispielsweise Bentonite, oder ein Gemisch aus Polyethylenglykol und Polyethylenglykolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

### O/W Emulsionen

| **Beispiele:** | **1** | **2** | **3** | **4** | **5** | **6** | **7** | **8** | **9** | **10** |
|---|---|---|---|---|---|---|---|---|---|---|
| Glycerinmonostearat SE | 0,50 | 1,00 | 3,00 | | | 2,50 | | | | |
| Glycerylstearat citrat | 2,00 | | | 1,00 | 2,00 | | 2,50 | | 3,00 | |
| Stearinsäure | | 3,00 | 0,75 | 2,00 | | | | | | |
| Sorbitanstearat | | | | | | | | | 1,00 | |
| Polyglycerylmethylglucosedistearat | | | | | | | | 3,00 | | |
| Ceylsttearyl Alkohol, PEG-40 Rizinusöl, Natrium Cetylstearyl Sulfate | | | | | | | | | | 4,00 |
| PEG-40 Stearat | 0,50 | | | | | 1,10 | | | | |
| PEG-100 Stearat | | | 1,50 | | | | | | | |
| Laurylmethiconcopolyol | | | | 0,75 | | 0,50 | | | | |
| Cetylphosphat | | | 0,75 | | 1,00 | | | | | |
| Stearylalkohol | | | 3,00 | | | 4,00 | 0,50 | 1,00, | | 2,00 |
| Cetylalkohol | 2,50 | 1,00 | | | 0,50 | | 2,00 | 1,00 | 2,00 | |
| Bis-Ethylhexyloxyphenol Methoxyphenyl Triazin | | | | | | | | | | 1,5 |
| Diethylaminohydroxybenzoylhexylbenzoat | | | 2,0 | | | | | | | |
| Titandioxid T 805 | | 2,0 | | | | | | | | |
| C12-15 Alkyl Benzoat | | 2,50 | | | | 7,00 | 5,00 | 3,00 | 6,00 | |
| Dicaprylylether | | | 3,50 | | 2,00 | | | 3,00 | 2,00 | |
| Butylenglycoldicaprylat/dicaprat | 5,00 | | | 5,00 | 3,00 | | | | 1,00 | 3,00 |
| Cetylstearylisononanoat | | 4,00 | | | | 2,00 | 2,00 | 2,00 | | |
| Dimethicon | | 0,50 | 1,00 | | 2,00 | | | 2,00 | | 2,00 |
| Cyclomethicon | 2,00 | | | 4,50 | | | 0,50 | | 3,00 | 3,00 |
| Dimethicon / Vinyl Dimethicon Crosspolymer | | 4,00 | | | | | 0,50 | | | |
| PVP Hexadecen Copolymer | 0,50 | | | 0,50 | 1,00 | | 1,00 | | | |
| Glycerin | 3,00 | 7,50 | | 7,50 | 5,00 | | 2,50 | 10,00 | 12,00 | 8,00 |
| Xanthangummi | 0,15 | | 0,05 | | | | 0,30 | | | |
| Natrium Carbomer | | | | | | | | 0,20 | 0,30 | 0,2 |
| Glycerin | | 5,00 | | | | 7,00 | | | | |
| Vitamin E Acetat | 0,50 | | 0,25 | 0,50 | 0,75 | | 1,00 | | 0,50 | 0,50 |
| Ubichinone | 0,25 | 0,10 | 0,05 | 0,20 | 0,003 | 0,10 | 0,04 | 0,10 | 0,15 | 0,08 |
| Glycin Soja | 0,05 | 0,30 | 1,50 | 1,00 | 1,00 | 2,00 | 1,8 | 1,00 | 3,00 | 0,9 |
| Creatine | 0,10 | | | 1,00 | | | 0,50 | | 0,30 | |
| 1-Methylhydantoin-2-imide | 0,01 | | | 0,40 | | | 0,20 | | 0,10 | |
| DMDM Hydantoin | | 0,60 | | 0,20 | | | | | 0,20 | |
| Methylparaben | 0,15 | | 0,25 | | 0,50 | 0,28 | | 0,20 | | 0,30 |
| Propylparaben | | | | | | 0,14 | | | | |
| Phenoxyethanol | 1,00 | 0,40 | | 0,40 | 0,50 | 0,50 | 0,60 | 0,50 | | 0,40 |
| EDTA | | 0,20 | 0,35 | 0,50 | 0,02 | | 0,03 | | | |
| Ethanol | | 2,00 | 1,50 | | 3,00 | 5,00 | 1,00 | | | |
| Parfüm | 0,20 | 0,20 | | | | 0,30 | 0,40 | | 0,25 | 0,30 |
| Wasser | ad 100 | ad 100 | ad 100 | ad 100 | ad 100 | ad 100 | ad 100 | ad 100 | ad 100 | ad 100 |

| | | | | | | | | | | |
|---|---|---|---|---|---|---|---|---|---|---|
| Glycin Soja = Isoflavon 150 der Fa. Lucas Meyer | | | | | | | | | | |

| SCHAUMFÖRMIGE O/W- EMULSIONEN: | | | | |
|---|---|---|---|---|
| | Beispiel 11 | | Beispiel 12 | |
| | GEW.-% | Vol.-% | GEW.-% | Vol.-% |
| Stearinsäure | 5,00 | | 1,00 | |
| Cetylalkohol | 5,50 | | | |
| Cetylstearylalkohol | | | 2,00 | |
| PEG-40 Stearat | 8,50 | | | |
| PEG-20-Stearat | | | 1,00 | |
| Caprylsäure/Caprinsäuretriglyceride | 4,00 | | 2,00 | |
| C12-15 Alkyl Benzoat | 10,00 | | 15,50 | |
| Cyclomethicon | 4,00 | | | |
| Dimethicon | | | 0,50 | |
| Octylisostearat | | | 5,00 | |
| Myristyl Myristat | | | 2,00 | |
| Ceresin | 1,50 | | | |
| Glycerin | | | 3,00 | |
| Phenybenzimidazol Sulfonsäure | | | 1,0 | |
| Butyl Methoxydibenzoylmethan | | | 2,0 | |
| Ethylhexyl Triazon | 1,5 | | | |
| BHT | | | 0,02 | |
| Na₂H₂EDTA | 0,50 | | 0,10 | |
| Ubichinon | 1,00 | | 0,20 | |
| Glycin Soja | 0,10 | | 0,80 | |
| Creatine | | | 0,80 | |
| 1-Methylhydantoine-2-imide | | | 0,30 | |
| Parfüm, Konservierungsmittel, | Q.S. | | Q.S. | |
| Farbstoffe, usw. | Q.S. | | Q.S. | |
| Kaliumhydroxid | Q.S | | Q.S | |
| Wasser | AD 100,00 | | AD 100,00 | |
| | pH-Wert auf 6,5-7,5 | | pH-Wert auf 5,0-6,0 | |

| **Emulsion 1** | | 70 | | |
|---|---|---|---|---|
| **Emulsion 2** | | | | 35 |
| Gas (Stickstoff) | | 30 | | |
| Gas (Helium) | | | | 65 |

Vereinigung der auf 78 °C aufgeheizten Fett-/Lichtschutzfilterphase mit der auf 75 °C aufgeheizten Wasser-/Lichtschutzfilterphase. Homogenisierung mittels einer Zahnkranzdispergiermaschine (Rotor-Stator-Prinzip) bei 65 °C. 45 min Rühren im Becomix unter Begasung mit Helium bei 1 bar unter Kühlung auf 30 °C. Zugabe der Additive bei 30 °C (Parfüm). Homogenisierung mittels einer Zahnkranzdispergiermaschine (Rotor-Stator-Prinzip) bei 23 °C.

## Patentansprüche

1. Verwendung kosmetischer oder dermatologischer Zubereitungen in Form von O/W-Emulsionen mit einem Gehalt an
(a) einem oder mehreren Biochinonen und
(a) einem oder mehreren Isoflavonoiden
zur Straffung und/oder Festigung der Haut.

2. Verwendung kosmetischer oder dermatologischer Zubereitungen in Form von O/W-Emulsionen mit einem Gehalt an
(a) einem oder mehreren Biochinonen und
(a) einem oder mehreren Isoflavonoiden
zur kosmetischen und/oder dermatologischen Behandlung und/oder Prophylaxe der Cellulite.

3. Verwendung nach Anspruch 1 oder 2, **dadurch gekennzeichnet, dass** als Biochinon das Coenzym Q10 gewählt wird.

4. Verwendung nach einem der vorstehenden Ansprüche, **dadurch gekennzeichnet**, bei dass als Isoflavonoid das Genistein gewählt wird.

5. Verwendung nach einem der vorstehenden Ansprüche, **dadurch gekennzeichnet, dass** das oder die Isoflavonoide als Isoflavonoid-haltiger Extrakt in Form eines Soja-Extraktes eingesetzt wird oder werden, der neben Isoflavonoiden, bevorzugt Genistein, 5 - 20 Gew.-% Saponine, besonders vorteilhaft 10 - 18 Gew.-% Saponine, jeweils bezogen auf das Gesamtgewicht des Extraktes, enthält.

6. Verwendung nach einem der vorstehenden Ansprüche, **dadurch gekennzeichnet, dass** in den fertigen Zubereitungen Konzentrationen von 0,000.001 - 5 Gew.-% an einem oder mehreren Biochinonen, bevorzugt Coenzym Q10, gewählt werden, bezogen auf das Gesamtgewicht der Zubereitungen.

7. Verwendung nach einem der vorstehenden Ansprüche **dadurch gekennzeichnet, dass** in den fertigen Zubereitungen Konzentrationen von 0.001 - 2 Gew.-% enthalten sind, bevorzugt 0,02 - 0.04 Gew.-%, an einem oder mehreren Saponinen, jeweils bezogen auf die Gesamtzusammensetzung der Zubereitungen.
